(19)

Europäisches
Patentamt
European
Patent Office
Office européen
des brevets

(11) **EP 3 838 143 A1**

(12) **EUROPEAN PATENT APPLICATION**

(43) Date of publication:
**23.06.2021 Bulletin 2021/25**

(51) Int Cl.:
**A61B 5/11** *(2006.01)* **A61H 3/02** *(2006.01)*
**A61B 5/0488** *(2006.01)* **A61B 5/00** *(2006.01)*

(21) Application number: **19383121.1**

(22) Date of filing: **16.12.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **Fundación Tecnalia Research & Innovation**
**20009 San Sebastián, Guipúzcoa (ES)**

(72) Inventor: **BARRALON, PIERRE**
**20009 San Sebastián - Guipúzcoa (ES)**

(74) Representative: **Balder IP Law, S.L.**
**Paseo de la Castellana 93**
**5ª planta**
**28046 Madrid (ES)**

(54) **MOBILITY DEVICE FOR MONITORING AND/OR REHABILITATING WALKING ACTIVITY**

(57)   The present invention relates to a mobility device for monitoring and rehabilitating walking activity comprising at least one shaft (**101**); a handle (**102, 32**) connected to each of the at least one shaft (**101**); at least one sensor (**103, 104**) in at least one of the shafts (**101**) to measure parameters related to the walking activity of a user; and a battery-powered electronic module (**105**) connected to the at least one sensor (**103, 104**) to receive and process data from the sensor. The device is suitable for monitoring daily walking activity and/or in rehabilitation after surgery or any kind of lower limb mobility disorders. The parameters assessed by the device provide the means for monitoring the recovery induced by a therapy. It can also be a tool to motivate independent elderly to walk by monitoring and giving them feedback on how much they walk every day.

**EP 3 838 143 A1**

## Description

Field of the invention

The present invention relates to the field of mobility devices for monitoring and/or rehabilitating walking activity. These devices are suitable for monitoring daily walking activity and/or in rehabilitation after surgery (hip, knee, ankle...) or any kind of lower limb mobility disorders. They can also be a tool to motivate independent elderly to walk by monitoring and giving them feedback on how much they walk every day.

Background of the invention

The extent of daily physical activity is of primary interest in rehabilitation medicine, it is a key determinant in the evaluation of quality of life, and is related to health (D. W. Brown et al. "Associations between recommended levels of physical activity and health-related quality of life. Findings from the 2001 behavioral risk factor surveillance system (brfss) survey". Prev Med, vol. 37, no. 5, pp. 520-528, Nov 2003 and K. F. Koltyn, "The association between physical activity and quality of life in older women". Womens Health Issues, vol. 11, no. 6, pp. 471-480, 2001). Walking is one of the most important daily physical activities. In fact it is a prerequisite to many other activities, and is often experienced by amputee patients as limited (C. Collin and J. Collin, "Mobility after lower-limb amputation". Br J Surg, vol. 82, no. 8, pp. 1010-1011, Aug 1995). Accurate monitoring of the amount ("quantity") and way (gait pattern or "quality") of walking is considered important (C. A. V. Bennekom, F. Jelles, and G. J. Lankhorst, "Rehabilitation activities profile: the icidh as a framework for a problem-oriented assessment method in rehabilitation medicine". Disabil Rehabil, vol. 17, no. 3-4, pp. 169-175, 1995; W. K. Brodzka, H. L. Thornhill, S. E. Zarapkar, J. A. Malloy, and L. Weiss, "Long-term function of persons with atherosclerotic bilateral below-knee amputation living in the inner city". Arch Phys Med Rehabil, vol. 71, no. 11, pp. 895-900, Oct 1990 and M. M. Torres and A. Esquenazi, "Bilateral lower limb amputee rehabilitation. A retrospective review". West J Med, vol. 154, no. 5, pp. 583-586). A reliable and valid measure of walking parameters can assist a clinician in his diagnosis, in choosing the most suitable therapy for a patient, in monitoring patient progress and in assessing the effects of a treatment (A. Geurts, T. Mulder, R. Rijken, and B. Nienhuis, "From the analysis of movements to the analysis of skills: bridging the gap between the laboratory and the clinic". Journal of Rehabilitation Sciences, vol. 4, pp. 9-12, 1991).

Gait analysis commonly involves the measurement of the movement of the body in space (kinematics) and the forces involved in producing these movements (kinetics). It can be measured using two types of techniques: non-ambulatory and ambulatory techniques.

Non-ambulatory techniques, such as photographies, video recordings, marker systems and force-plates (which measure the ground reaction force including both magnitude and direction) are not suitable for monitoring such activities continuously during daily life.

Ambulatory techniques, on the other hand, refer to techniques that can be used by the patient during daily life and don't need him/her to stay in hospital. Ambulatory approaches can be subdivided into four classes: devices attached to the body, embedded into the jacket, embedded into the shoes and embedded into the canes.

Canes improve balance and gait, and also benefit to people who have had a stroke. However Hamzat (T. Hamzat and A. Kobiri, Effects of walking with a cane on balance and social participation among community-dwelling post-stroke individuals," Eur J Phys Rehabil Med, vol. 44, pp. 121{6, 2008.) warns that in planning a rehabilitation program for post-stroke hemi-paretic individuals, the use of a walking device to aid ambulation should be critically considered. This is because using a cane (mobility device) tends to have a negative impact on social participation. This funding enhances the need of monitoring continuously the rehabilitation process in order to make it more efficient and therefore stop using the mobility assistive device as soon as possible. Therefore, the integration of sensors into the cane (mobility device) is a solution.

US Patent 6011481 refers to a walking cane with sensors which includes sensors into the multiple legs and gives feedback to the user about an imbalance in the weight distribution among the multiple legs of the cane, indicative of an approaching unstable situation. The user can then stop and probe with the cane until a stable position is found.

US Patent 6668846 B2: discloses a walking cane which substantially maintains its position despite attempts to move or deflect the cane, by means of a gyroscope. However, this cane does not provide any information on walking parameters of the user.

JP 2006085609: discloses an elderly person watching system, wherein the walking stick detects contact with a user, contact with the ground, stick acceleration and stick inclining degree by a built-in sensor, and determines whether the user is normal or abnormal on this information. When the user is normal, safety information is provided to a watching person, and when the user is abnormal, emergency information is provided to the watching person.

US Patent 7066864 B2: relates to a walking pole used in Nordic walking or skiing. The tip part is connected to a power sensor and gives information to the user about the exercise performance.

EP 1908499 A1 relates to a sport stick with sensor enhancements. The piece of sports equipment comprises sensors capable to measure acceleration, speed, angles, position, altitude, humidity and temperature which can generate visual, acoustic and/or vibration signals, providing the user with an immediate feedback of his training.

However, all of the previous devices are using 1D, 2D or 3D load cells or even 6 DoF force sensor (3 forces, 3 moments), which are very expensive components. Moreover, the ICP components require an excitation voltage between 20V and 30V, which is not suitable for an ambulatory apparatus.

US 20160300469 A1 describes an ultrasonic distance sensor attached to an assistant and pointing forwards and downwards. The ultrasonic distance sensor sensing an approaching drop in elevation of a ground surface such as an open staircase leading downwards.

JP2017042251-A describes a walking stick for assisting a handicaped person. The stick comprises a sensor for detecting the distance of an approaching obstacle.

CN207506751U describes a medical walking stick with alarming system comprising a distance sensor such that when in a sudden situation the crutches are dropped on the ground, and the distance between the sensor 5 and the ground is instantaneously reduced, the distance sensor transmits this situation a the control chip and triggers an alarm.

US-2012029696-A1 describes a robotic cane comprising sensors to obtain a balance signal corresponding to the orientation of the cane.

None of these patents serves for the rehabilitation of the walking activity

Therefore, a need still exists in the art of a mobility device for monitoring walking activity which is low-priced and user-friendly, which can be used in an ambulatory setting by a patient. Moreover, this device should provide information on parameters related to the walking activity of a user, and not only related to the weight distribution thereon.

Summary of the invention

The present invention relates to a mobility device, such as a cane, a walker, a crutch or hiking pole, for monitoring or rehabilitating walking activity by making use of at least one sensor to measure parameters related to the walking activity of a user. The device is modular allowing different degrees of system complexity/functionality to be achieved. The mobility device comprises

- at least one shaft with a handle to hold the shaft and a base provided to touch the ground when the person is walking using the device;
- at least one sensor in at least one of the shafts to measure parameters related to the walking activity of the person and mounted on the shaft so that during walking the cited sensor is pointing toward the ground to measure a first distance between the sensor and the ground.

The mobility device also comprises an electronic module connected to the at least one sensor to receive and process data from the sensor.

The mobility device can comprise an additional sensor mounted on the shaft so that during walking the cited additional sensor is pointing toward the ground to measure a second distance between the additional sensor and the ground.

In a first embodiment the sensor and the additional sensor are comprised in a sagittal plane to estimate a sagittal angle formed between the mobility device and a vertical direction and to estimate the shaft's elevation from the ground.

In a second embodiment the sensor and the additional sensor are comprised in a coronal plane to estimate a coronal angle formed between the mobility device and a vertical direction and to estimate the shaft's elevation from the ground.

The mobility device can comprise at least one leg sensor mounted on the shaft so that during walking the cited leg sensor is pointing towards one of the legs of the user to measure a distance between the leg sensor and the leg of the user.

The leg sensor can be mounted on the shaft to point to the leg closer to the shaft when the user holds the shaft with one hand and the mobility device further comprises an additional leg sensor mounted on the shaft in opposition to the leg sensor and pointing towards the legs of the user to measure the distance between the additional sensor and the legs, when the user holds the mobility device with the other hand.

The sensors can be selected from the group consisting of ultrasonic sensors, infrared sensors and laser.

The electronic module can be powered by either a battery or an energy scavenging module or a combination of both.

The mobility device can comprise a data storage unit and an interface/display so the user can monitor his/her own activity.

The electronic module can communicate with an auxiliary system for further processing.

## Brief description of the drawings

The present invention will be better understood by reference to the following drawings illustrating preferred embodiments of the invention, provided by way of example, and which should not be construed as limiting the invention in any way.

Figure 1 shows a front view of a preferred embodiment of the mobility device of the present invention.

Figure 2 shows a perspective view of another preferred embodiment of the mobility device of the present invention.

Figure 3 is a diagrammatic view of the positioning of the sensors relative to the ground, the legs, and anatomical planes of the user according to the embodiment shown in Figure 1

Figure 4 is a diagrammatic view of different situations: initial contact, elevation, rotation. And the measurement obtained by one sensor 103 of the present invention according to the embodiment shown in Figure 1.

Figure 5 is a diagrammatic view similar to that shown in Figure 4, according to another preferred (two sensors 103) embodiment of the mobility device of the present invention.

Figure 6 presents a block diagram of the electronic module 105.

Figure 7 is a graph showing an example of recorded data using one preferred embodiment shown in Figure 1. Distances between the ground and two 103 sensors as well as the distance between the sensor 104 and any object in its cone of detection (mainly the two legs) is depicted in function of the time.

Figure 8 present the notations used to calculate the angle and elevation of the shaft using two sensors (103 and 103') or (113 and 113').

Figure 9 is a graph showing the estimated sagittal angle ($\theta$s) of the shaft relative to the vertical in function of the time and according to the mounting shown in Figure 5.

Figure 10 is a graph showing the estimated elevation relative to the ground level in function of the time, for the mounting shown in Figure 5.

Figure 11 presents the situation where an obstacle stands in front of the mobility device.

Figure 12 shows a 2-D plane constructed by the two distances measured by sensors **103** and **103'** (Figure 5), for obstacle detection.

## Detailed description of the preferred embodiments

The present invention relates to a mobility device for monitoring and/or rehabilitating walking activity comprising: at least one shaft **101;** a handle **102** connected to each of the at least one shaft; at least one sensor in at least one of the shafts to measure parameters related to the walking activity of a user; an electronic module **105** connected to the sensor(s) to receive and process data. This electronic module is powered by either a battery or an energy scavenging module (inertial microsystem, electroactive materials among others) or a combination of both. The sensor(s) provided in the mobility device according to the present invention can be of any suitable kind, preferably ultrasonic, infrared, lasers or other range sensors.

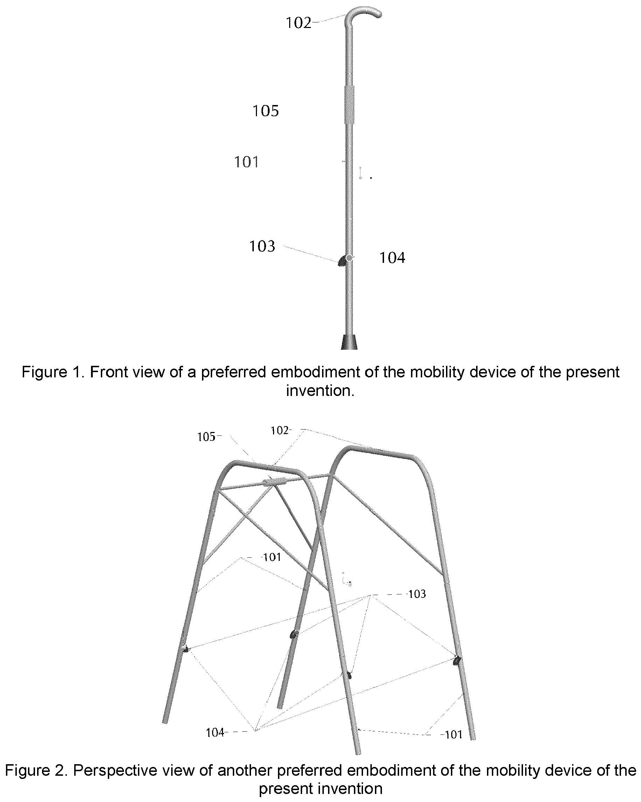

A preferred embodiment of the present invention is shown in Figure 1, which describes a cane as a mobility device. It comprises a vertical shaft **101,** equipped with a handle **102** and a base or foot. The cane is equipped with a sensor **103** pointing down to the ground and therefore measuring a first distance (d) from the sensor **103** to the ground. A leg sensor **104** is pointing toward the legs 305, 306 of the subject to measure the distance 302 between the sensor **104** and any object in its cone of detection but mainly the legs 305, 306 of the user. The sensors **103, 104** are not necessarily located at the same distance from the ground, although this is substantially the case shown in the embodiment of Figure 1. Both sensors **103, 104** are connected to an electronic module **105,** mounted on or in the shaft **101** of the cane, by way of wires that may be carried inside or outside the cane shaft **101.** The features of the electronic module **105** will be further described herein below.

In a preferred embodiment, in the cane shown in Figure 1 (as well as in other similar embodiments such as a crutch and the like) the sensor **104** directed toward the legs of the user can be located on either side of the shaft **101** for being used by left-handed and right-handed people. In a further preferred embodiment (not shown in the Figures), an additional leg sensor **104'** can be provided in the shaft **101,** on the opposite side of the leg sensor 104, allowing the user to change the hand which holds the mobility device.

Figure 2 shows another embodiment of the present invention. In Figure 2, like elements are designated with the same numerals as in Figure 1. A walker is shown which comprises four shafts **101,** two front and two rear shafts. Each front shaft is connected by a handle **102** to its corresponding rear shaft. As can be shown in this embodiment, each shaft **101** is equipped with one sensor **103** and one sensor **104,** pointing toward the ground and to the legs of the user, respectively. The electronic module **105** is mounted on a cross member joining the two front shafts or wherever it is the less disturbing.

The above Figures 1 and 2 have shown two different embodiments of the present mobility device: a cane and a walker, respectively. However, other modifications will be apparent to those skilled in the art to implement the present invention, such as for example a crutch a hiking pole and the like, and are intended to be within the scope of the present invention. Although the walker shown in Figure 2 includes a total of eight sensors (four sensors **103** and four sensors **104**), in another embodiment it will include a different number of sensors **103** and/or **104** without departing from the spirit of the invention.

Although the mobility devices shown in the Figures 1 and 2 relating to preferred embodiments of the present invention include both sensors **103** and **104,** those skilled in the art will easily understand that sensors **103** and **104** can be of different kind (e.g. 103: ultrasonic, 103': infrared, 104: laser, and so on) without departing from the scope thereof.

The following will describe the operation of the sensors **103, 104** and the electronic module **105** for the cane shown in Figure 1. However, this operation will be analogous in any other embodiment of the mobility device (such as a walker, a crutch, hiking poles and the like).

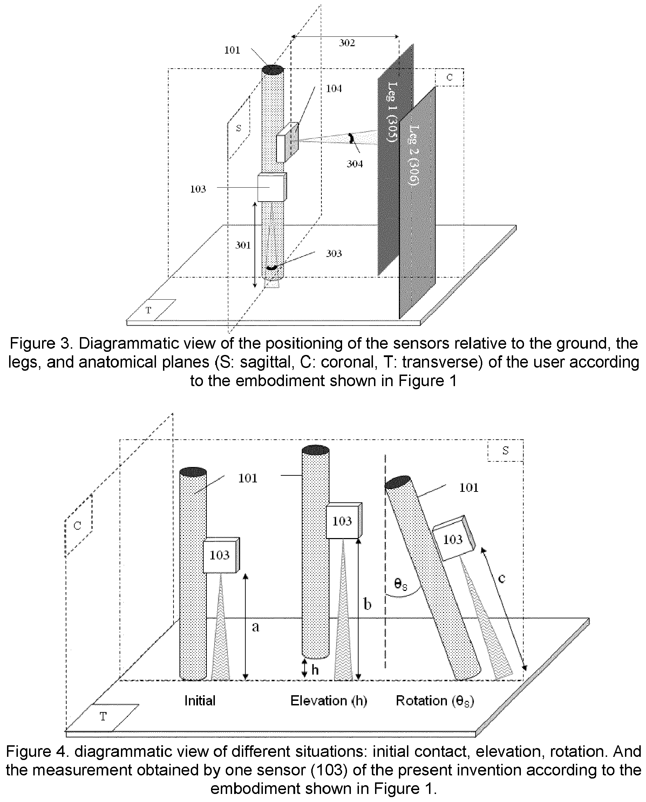

A more complete understanding of the invention and its advantages will be apparent by consideration of Figure 3, which shows the positioning of the sensors **103, 104** relative to the ground and the legs of the user, respectively. This kind of sensor measures the distance to the closest object in its beam. The sensor **103** is pointing toward the ground which means that its positioning may or may not generate an initial angle in the sagittal (S) and/or coronal (C) planes. A sagittal plane of the mobility device is an imaginary plane that travels vertically from the top to the bottom of the device dividing it into left and right portions. The coronal plane divides the mobility device into anterior and posterior portions. The sensor **103** has a beam angle **303** that can be adjusted for different application purposes. The sensor **104** is pointing toward the legs of the user with a beam angle **304** that can be adjusted for different application purposes. Its positioning may or may not generate an initial angle in the sagittal and/or coronal planes. As previously explained, they are both connected to an electronic module **105** by way of wires that may be carried inside the shaft **101.** The electronic module **105** comprises a means for accessing the data provided thereby. This means may be a feedback interface **612** or **614** for the user to monitor his/her own activity, a communication unit with a remote system, or a combination of both. Alternatives of the means for accessing the data will be further described hereinbelow. In one embodiment, the electronic module **105** may itself be placed entirely within shaft **101.** The electronic module may be a hardware logic unit to process the data sent by the sensors **103, 104** according to an algorithm as will be explained hereinbelow.

In use, the sensor **103** measures a first distance (d) from its location to the ground **301** and communicates the data continuously to the electronic module **105.**

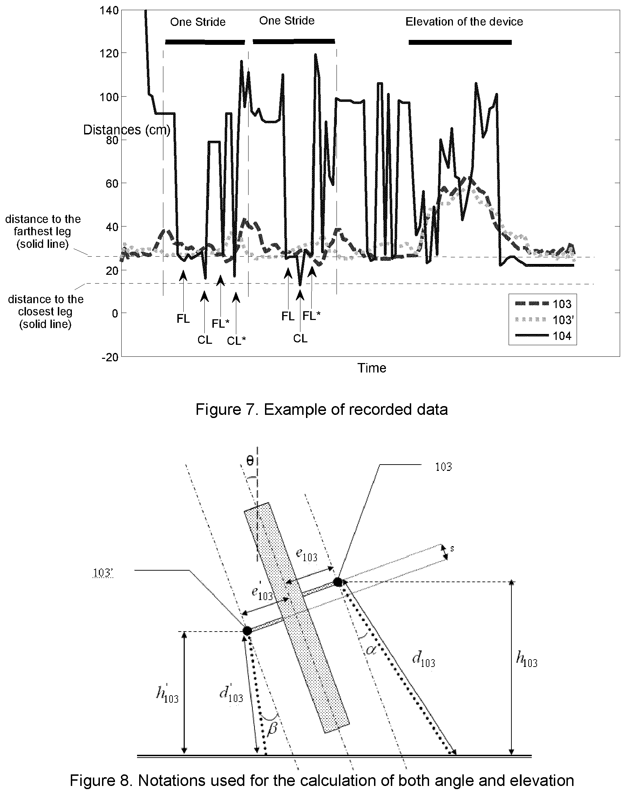

In use, the sensor **104** measures the distance 302 from its location to the closest leg **305** and communicates the data continuously to the electronic module **105.** The electronic module **105** collects information about the relative movements of the legs around the shaft **101** and therefore gives information about the gait. For example, in the case of a cane, in a typical situation the shaft **101** follows the opposite leg **306.** Therefore the sensor **104** detects whenever the leg **305** passes in front of the sensor **104** (see also Figure 7).

Figure 4 further represents the operation of a single sensor **103** to measure a first distance (d) to the ground (a, b, and c) and estimate the angle with the vertical $\theta$s. By this measurement it can detect when the shaft **101** of the mobility device is in a vertical position and touching the ground: d equals an initial value, a. Then whenever the walker begins a new step by lifting the mobility device and moving it ahead, the new value of (d) (i.e. b or c) will be greater than a. Every time that (d) will be equal to a, this will indicate the end of a cycle including two steps. Moreover, (d) gives information on the use of the mobility device. Indeed, if the subject is walking with the mobility device always in a vertical position, then (d) fluctuates between a and b (with max(|b-a|) around 10 cm). This scenario corresponds to subjects making short steps. Otherwise when making longer steps, the mobility device is placed further forward. This is usually done by rotating the mobility device around the wrist on the sagittal plane, moving it forward and putting it down. The rotation applied to the mobility device implies a measurement of (d) much bigger (c). Knowing the location of the sensor on the shaft **101**, and under the hypothesis that there is no object in front of the user, the sagittal angle ($\theta$s) with the vertical can be estimated.

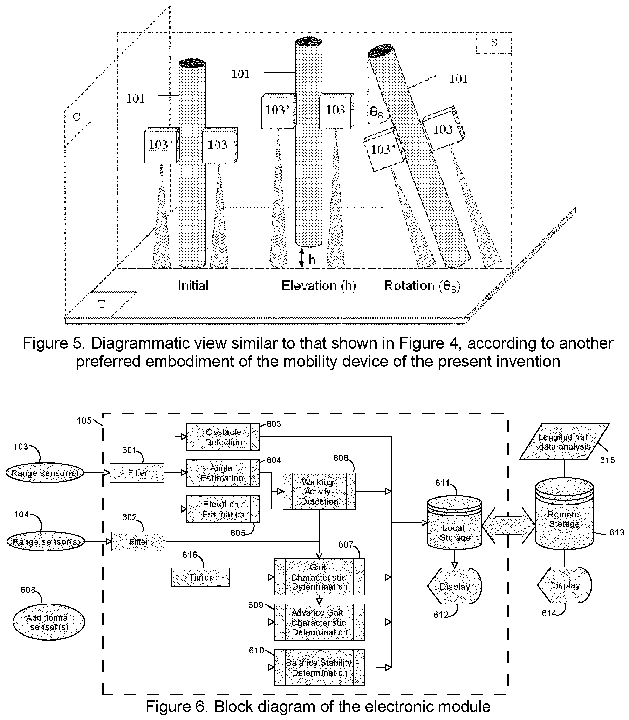

Figure 5 shows another preferred embodiment of the present invention in which the mobility device includes two sensors **103** pointing toward the ground. This additional sensor **103'** greatly facilitates the estimation of both elevation (h) of the mobility device and the sagittal angle ($\theta$s) when walking (equation 1) .

Figure 6 is a general block diagram of the mobility device for monitoring and/or rehabilitating walking activity. Data from the range sensors **103** and **104** may be filtered by the filters **601** and **602** respectively. The type and characteristics of the two filters may or may not be different. For example, a maximally flat response Infinite Impulse Response (IIR) low-pass filtering algorithm with a cut-off frequency of 3Hz may be used. In some implementations, in order to eliminate the phase shift caused by filtering, a non-causal bi-directional filter may be used. In other implementations, a causal filter with minimal phase shift may be used, or a causal filter with a linear phase response with a minimal order necessary to guarantee a desired on-line processing speed may be used. Other filter types, whether implemented in software or hardware, also may be used. For example, Finite Impulse Response (FIR) filters or Butterworth HR filter may be used. A magnitude response of the Butterworth HR filter may be designed to be flat, and the phase response may be designed to be approximately linear in the pass-band. With these characteristics and lower order than the corresponding FIR filter, a high order Butterworth filter may be implemented for on-line gait detection. A minimum phase shift Butterworth HR filter for on-line gait characterization also may be used.

Raw of filtered from may be analyzed to estimate both the angle **604** and elevation **605** of the shaft **101.** Range data recorded from the sensor(s) **103** may include cyclic or quasi-cyclic changes reflecting a cycle of the mobility device, and, from such changes, walking activity may be detected **606** from other movements. When a walking activity period is detected, data provided by sensor (s) **104** may be used in conjunction with timing information from a timer **616** to determine the basic gait characteristics **607,** such as number of steps per minute, duration of each step. At the same time the data for the sensor (s) **103** may be analyzed to detect obstacle **603.**

Additional information from accelerometers and/or gyroscopes and/or magnetometers and/or pressure and /or torque sensor(s) **608** mounted onto the mobility device may be used in conjunction with the gait characteristic determination **607,** in order to perform an advanced gait characteristic determination **609.** The advanced gait characteristic determination **609** may include, for example, a determination of an instantaneous or average walking velocity of the user, step length of the user, stride length of the user, force applied on the mobility device. Additionally, or alternatively, a balance and stability determination **610** may be made. For example, forces and moments applied to the mobility device and measured at the handle **102** may be used in conjunction with the orientation of the shaft(s) **101** and the distance **302** for the legs to the shaft(s) to estimate body's sway using rigid body transformations. As a result, an index of stability of the mobility device and/or user may be evaluated. Local storage **611** is used to store the sensor(s) data and/or walking detection period and/or basic **607** and advanced **608** gait characteristics and/or balance characteristics **610** and/or obstacle detection **603.**

The means for accessing the data provided by the electronic module **105** may be a display **612,** typically a visual display (but audio, haptic, or a combination of these modalities could be used) so that the user can monitor his/her own activity or can be warn in case of an obstacle detected. The contents of the local storage **611** may also be transferred by a wired and/or wireless communication unit to a remote storage **613,** for being display **614** to professionals or informal carers or for longitudinal data analysis **615.**

When the mobility device is equipped with a wired and/or wireless communication unit, an auxiliary system is provided that remotely communicates with the mobility device trough a network protocol. This auxiliary system is designed to be used by medical staff (or informal carers) and to provide processing and data storage **613** means together with proper software to analyze and display **614** the data collected and sent by the mobility device during the rehabilitation or telerehabilitation sessions.

For the purpose of establishing the initial degree of impairment and to monitor patient improvement with a therapy, the measurement method described hereinabove is implemented. As previously explained, the method relies on the measurement and recording of the movement of the mobility device regarding the ground and/or the legs of the subject. The rehabilitation training method takes advantage of a software that interactively instructs the patient about the task to carry on by using the mobility device. The patient continuously receives feedback about his/her performance in a sort of interactive game. The type of exercise to be performed can be decided remotely by care center staff and can constantly be transmitted to the user in order to improve the recovery.

The complete system is modular, so the embodiments previously described can be integrated by add-ons to extend its functionality. Examples of possible add-ons to the mobility device of the present invention will now be provided.

In an embodiment, EMG monitoring of the lower limb muscles can be added in order to provide the medical staff with more data about muscle activations so that the therapy can adjusted accordingly.

In another embodiment, there is a set of low resolution CMOS or CCD cameras that can be used to video monitor the training movements qualitatively and/or quantitatively (trajectories and/or angular positions of the lower limb).

The above description of the preferred embodiments is not to be construed as limiting, but rather descriptive of the present invention, and these and other modifications of the described embodiments that may occur to those persons skilled in the art are intended to be comprised within the scope of the present invention.

As an example of data collected by the one embodiment (Figure 1), Figure 7, shows the distance measured between two sensors (103 and 103') and the ground, and between one sensor 104 and the legs of the user, respectively, in function of the time. Each peak on the sensor **103** curve represents the maximum distance to which the sensor **103** is taken from the ground. On this figure these times are used to illustrate gait cycle or stride. The curve corresponding to the sensor **104** represents the distance between said sensor and the legs of the user, indicating every time that the Farthest Leg (FL) and the Closest Leg (CL) pass in the cone detection of the sensor **104.** Whenever two steps are completed, the walking stick (in this embodiment) moves forward to initiate a new stride. During this movement the sensor(s) **104** may detect one or the two legs. This detection is noted by an asterisk on Figure 7 (FL* and CL*). These detections give information about the gait of the user (for example the cadence, step duration, stride duration).

Figure 8 shows a general situation of the mobility device where two sensors **(103, 103')** off-centred (e,,e') from the shaft with initials orientations ($\alpha$, $\beta$). This estimation of the angle and elevation is done using this initial system of equations (1):

$$\begin{cases} d(\theta,\alpha).\cos(\theta+\alpha) = h(\theta,\alpha) \\ d'(\theta,\alpha).\cos(\theta-\beta) = h'(\theta,\alpha) \end{cases} \quad (1)$$

Where $\theta$ and $h_{103}$ are two unknowns. d and d' are the distances measured from sensors 103 and 103' respectively. $\alpha$ and $\beta$ are the known initial orientations of sensors **103** and **103'**. h and h' are the vertical distances from sensors 103 and 103' to the ground 301 respectively. $h'_{103}$ can be expressed as a function of h (equation 2)

$$h' = h - (e'+e).\sin(\theta) - s.\cos(\theta) \quad (2)$$

When $\theta$ and h are calculated, and knowing the dimensions of the mobility device, the elevation of any parts of the mobility device can be assessed.

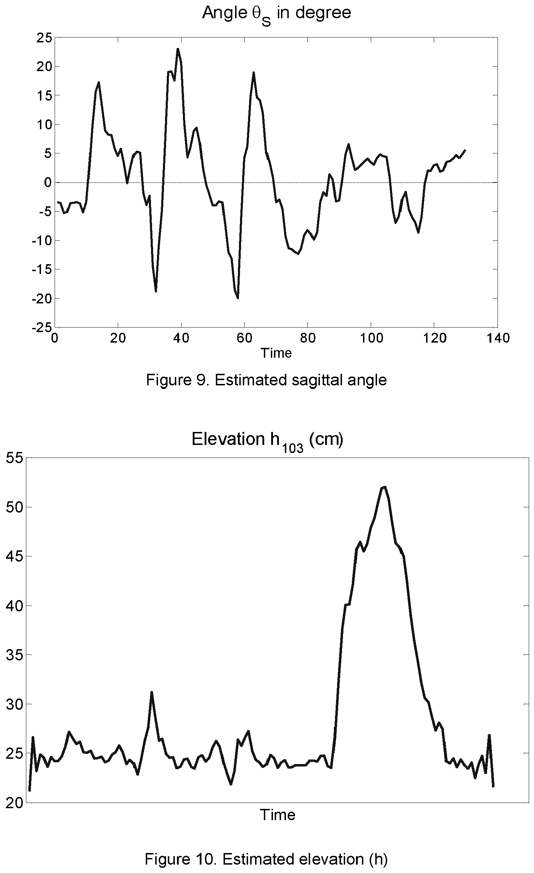

Figure 9 shows the sagittal angle ($\theta$s) estimated form the data presented on the Figure 7. Cyclic or quasi-cyclic changes can be observed and detected **606** when the subject is walking using frequency, time-frequency, time-scale, and/or peak detection methods for example.

Figure 10 shows the elevation (h) estimated form the data presented on the Figure 7. Low amplitude oscillations can be seen when the subject is walking and the elevation of the mobility device is correctly estimated at the end of the trail when the subject lifted up vertically the cane.

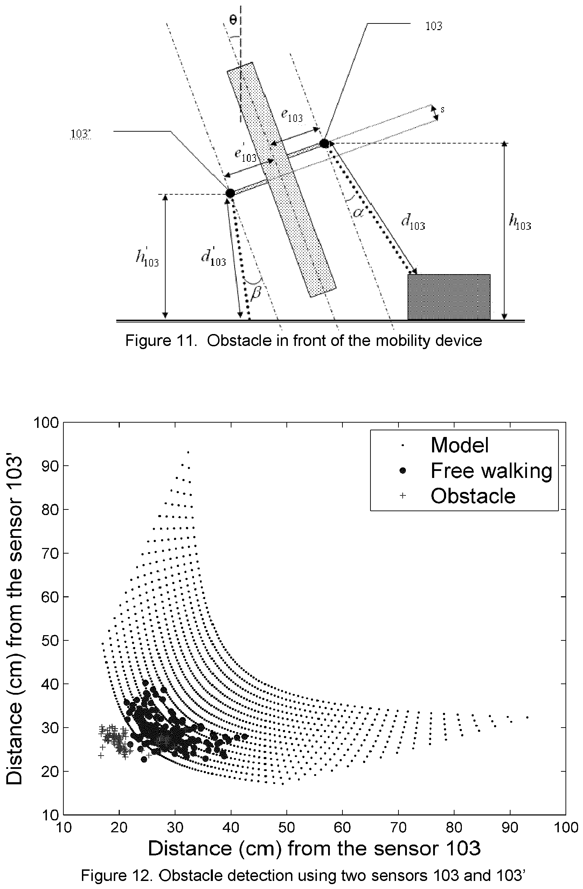

Knowing the initial positioning of the two sensors **103** and **103'** on the shaft **101** (Figure 8), typical values of $d_{103}$ and d' can be predicted by our model of the system (equations (1)). The predicted data are plotted on the Figure 12 and are labeled "Model". Data recorded during a free walking are also plotted and labeled "Free walking". Data recorded when close to an obstacle (Figure 11) are labeled "Obstacle". Obstacle detection depends on the positioning of the sensors **103** and **103'**. An initial angle between the sensors **103** and the shaft **101** (i.e $\alpha$ on Figure 11) is preferred.

**Claims**

1. A mobility device for monitoring and rehabilitating walking activity of a person comprising

   - at least one shaft (**101**) with a handle (**102**) to hold the shaft (**101**) and a base provided to touch the ground when the person is walking using the device;
   - at least one sensor (**103**, 103', 113, 113', **104, 104'**) in at least one of the shafts (**101**) to measure parameters related to the walking activity of the person; and
   - an electronic module (**105**) connected to the at least one sensor (**103**, 113) to receive and process data from the sensor, **characterized in that** the, at least one, sensor (**103, 113**) is mounted on the shaft so that during walking the cited sensor (103, 113) is pointing toward the ground to measure a first distance (d) between the sensor and the ground.

2. The mobility device according to claim 1, comprising an additional sensor (**103', 113'**) mounted on the shaft so that during walking the cited additional sensor (103', 113') is pointing toward the ground to measure a second distance (d') between the additional sensor (103',113') and the ground.

3. The mobility device according to claim 2, wherein the sensor (103) and the additional sensor (103') are comprised in an sagittal plane (S) to estimate an sagittal angle ($\theta_s$) formed between the mobility device and a vertical direction and to estimate the shaft's elevation (h, h') from the ground.

4. The mobility device according to claim 2, wherein the sensor (113) and the additional sensor (113') are comprised in a coronal plane (C) to estimate a coronal angle ($\theta_c$) formed between the mobility device and a vertical direction and to estimate the shaft's elevation (h, h') from the ground.

5. The mobility device according to claim 1, comprising at least one leg sensor (104) mounted on the shaft so that during walking the cited leg sensor (104) is pointing towards one of the legs (305, 306) of the user to measure a distance between the leg sensor (104) and the leg of the user.

6. The mobility device according to claim 5, wherein the leg sensor (104) is mounted on the shaft (101) to point to the leg closer to the shaft (101) when the user holds the shaft (101) with one hand, the mobility device further comprising an additional leg sensor (104') mounted on the shaft (101) in opposition to the leg sensor (104) and pointing towards the legs **(305, 306)** of the user to measure the distance between the additional sensor (104') and the legs, when the user holds the mobility device with the other hand.

7. The mobility device according to claim 2, wherein the two measurements of the sensor (103) and additional sensor (103') allow the detection of obstacle.

8. The mobility device according to any of previous claims, wherein the sensors (103, 103', 113, 113', 104, 104') are selected from the group consisting of ultrasonic sensors, infrared sensors and laser.

9. The mobility device according to any of previous claims, wherein the electronic module (105) is powered by either a battery or an energy scavenging module or a combination of both.

10. The mobility device according to any of previous claims, further comprising a data storage unit (611).

11. The mobility device according to any of previous claims, further comprising an interface/display (612) so the user can monitor his/her own activity.

12. The mobility device according to any of previous claims, wherein the electronic module (**105**) can communicate with an auxiliary system for further processing.

13. The mobility device according to claim 12, wherein the communication is a wireless communication.

14. The mobility device according to any of previous claims, wherein the device is selected from the group consisting of a cane, a walker, a crutch, and hiking pole.

15. The mobility device according to any of previous claims, for use to monitor and/or rehabilitate the walking activity.

102—

105

101

103

104

Figure 1. Front view of a preferred embodiment of the mobility device of the present invention.

105—    102-

101

103

104- - -    -101

Figure 2. Perspective view of another preferred embodiment of the mobility device of the present invention

Figure 3. Diagrammatic view of the positioning of the sensors relative to the ground, the legs, and anatomical planes (S: sagittal, C: coronal, T: transverse) of the user according to the embodiment shown in Figure 1

Figure 4. diagrammatic view of different situations: initial contact, elevation, rotation. And the measurement obtained by one sensor (103) of the present invention according to the embodiment shown in Figure 1.

Figure 5. Diagrammatic view similar to that shown in Figure 4, according to another preferred embodiment of the mobility device of the present invention

Figure 6. Block diagram of the electronic module

Figure 7. Example of recorded data

Figure 8. Notations used for the calculation of both angle and elevation

Angle $\theta_S$ in degree

Figure 9. Estimated sagittal angle

Elevation $h_{103}$ (cm)

Figure 10. Estimated elevation (h)

Figure 11. Obstacle in front of the mobility device

Figure 12. Obstacle detection using two sensors 103 and 103'

**EP 3 838 143 A1**

Europäisches
Patentamt

European
Patent Office

Office européen
des brevets

# EUROPEAN SEARCH REPORT

Application Number

EP 19 38 3121

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NAVE CARLOS ET AL: "Physical Rehabilitation based on Smart Walker", 2018 12TH INTERNATIONAL CONFERENCE ON SENSING TECHNOLOGY (ICST), IEEE, 4 December 2018 (2018-12-04), pages 388-393, XP033493579, DOI: 10.1109/ICSENST.2018.8603660 * figures 1-2 * * page 389 * title; * abstract * | 1-4,7-15 | INV. A61B5/11 A61H3/02 ADD. A61B5/0488 A61B5/00 |
| X | NAVE CARLOS ET AL: "Smart Walker based IoT Physical Rehabilitation System", 2018 INTERNATIONAL SYMPOSIUM IN SENSING AND INSTRUMENTATION IN IOT ERA (ISSI), IEEE, 6 September 2018 (2018-09-06), pages 1-6, XP033448846, DOI: 10.1109/ISSI.2018.8538210 * figures 1-2 * * page 2 * | 1-4,7-15 | |
| X | MASAHIRO YAMAMOTO ET AL: "Improvement of Road Surface Discrimination Performance of Movement Support System Using Ultrasonic Sensors", APPLIED COMPUTING AND INFORMATION TECHNOLOGY, ACM, 2 PENN PLAZA, SUITE 701NEW YORKNY10121-0701USA, 29 May 2019 (2019-05-29), pages 1-6, XP058442390, DOI: 10.1145/3325291.3325388 ISBN: 978-1-4503-7173-5 * figures 2, 4, 5 * * page 186 - page 187 * | 1-4,7-15 | TECHNICAL FIELDS SEARCHED (IPC) A61B A61H |

-/--

~~The present search report has been drawn up for all claims~~

| Place of search | Date of completion of the search | Examiner |
|---|---|---|
| The Hague | 8 July 2020 | Knoop, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number
EP 19 38 3121

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2016/300469 A1 (HOOD MARY E [US]) 13 October 2016 (2016-10-13) * figure 1 * * paragraph [0013] - paragraph [0018] * | 1-4,7-15 | |
| X,D | CN 207 506 751 U (WANG HUAIYUAN) 19 June 2018 (2018-06-19) * figure 1 * | 1-4,7-15 | |

TECHNICAL FIELDS SEARCHED (IPC)

~~The present search report has been drawn up for all claims~~

| Place of search | Date of completion of the search | Examiner |
|---|---|---|
| The Hague | 8 July 2020 | Knoop, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

| | Europäisches Patentamt<br>European Patent Office<br>Office européen des brevets | Application Number |
|---|---|---|
| | | EP 19 38 3121 |

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-4, 7-15

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 19 38 3121

The Search Division considers that the present European patent application does not comply with the requirements of unity of invention and relates to several inventions or groups of inventions, namely:

```
1. claims: 1-4, 7-15

    Improving the detection of the elevation of the mobility
    device. This is achieved by including a second sensor for
    distance measurement to the ground.
                                   ---

2. claims: 5, 6

    Detecting the user in relation to the mobility device. This
    is achieved by implementing a leg sensor to detect the
    distance to the leg of a user.
                                   ---
```

## ANNEX TO THE EUROPEAN SEARCH REPORT
## ON EUROPEAN PATENT APPLICATION NO.

EP 19 38 3121

This annex lists the patent family members relating to the patent documents cited in the above-mentioned European search report.
The members are as contained in the European Patent Office EDP file on
The European Patent Office is in no way liable for these particulars which are merely given for the purpose of information.

08-07-2020

| Patent document cited in search report | | Publication date | Patent family member(s) | Publication date |
|---|---|---|---|---|
| US 2016300469 | A1 | 13-10-2016 | NONE | |
| CN 207506751 | U | 19-06-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6011481 A
- US 6668846 B2
- JP 2006085609 B
- US 7066864 B2
- EP 1908499 A1
- US 20160300469 A1
- JP 2017042251 A
- CN 207506751 U
- US 2012029696 A1

### Non-patent literature cited in the description

- **D. W. BROWN et al.** Associations between recommended levels of physical activity and health-related quality of life. Findings from the 2001 behavioral risk factor surveillance system (brfss) survey. *Prev Med,* November 2003, vol. 37 (5), 520-528
- **K. F. KOLTYN.** The association between physical activity and quality of life in older women. *Womens Health Issues,* 2001, vol. 11 (6), 471-480
- **C. COLLIN ; J. COLLIN.** Mobility after lower-limb amputation. *Br J Surg,* August 1995, vol. 82 (8), 1010-1011
- **C. A. V. BENNEKOM ; F. JELLES ; G. J. LANKHORST.** Rehabilitation activities profile: the icidh as a framework for a problem-oriented assessment method in rehabilitation medicine. *Disabil Rehabil,* 1995, vol. 17 (3-4), 169-175
- **W. K. BRODZKA ; H. L. THORNHILL ; S. E. ZARAPKAR ; J. A. MALLOY ; L. WEISS.** Long-term function of persons with atherosclerotic bilateral below-knee amputation living in the inner city. *Arch Phys Med Rehabil,* October 1990, vol. 71 (11), 895-900
- **M. M. TORRES ; A. ESQUENAZI.** Bilateral lower limb amputee rehabilitation. A retrospective review. *West J Med,* vol. 154 (5), 583-586
- **A. GEURTS ; T. MULDER ; R. RIJKEN ; B. NIENHUIS.** From the analysis of movements to the analysis of skills: bridging the gap between the laboratory and the clinic. *Journal of Rehabilitation Sciences,* 1991, vol. 4, 9-12
- **T. HAMZAT ; A. KOBIRI.** Effects of walking with a cane on balance and social participation among community-dwelling post-stroke individuals. *Eur J Phys Rehabil Med,* 2008, vol. 44, 121